# EUROPEAN PATENT SPECIFICATION

(11) **EP 2 044 911 B1**
(45) Date of publication and mention of the grant of the patent: **20.04.2011**
(21) Application number: 08163441.2
(22) Date of filing: 01.09.2008
(51) Int. Cl.: A61F 9/011

(54) **Flexible surgical probe**
Flexible chirurgische Sonde
Sonde chirurgicale flexible

(30) Priority: 04.10.2007 US 867302
(43) Date of publication of application: 08.04.2009
(73) Proprietor: Alcon, Inc., 6331 Hünenberg (CH)
(72) Inventor: Auld, Jack R., Laguna Niguel, CA 92677 (US); Zica, Michael A., Costa Mesa, CA 92626 (US); Farley, Mark, Laguna Niguel, CA 92677 (US)
(74) Representative: Hanna, Peter William Derek

(56) References cited:
- EP-A2- 1 447 064
- WO-A2-2005/107664
- US-A- 3 858 577
- US-A- 5 281 214
- US-A- 5 416 878
- US-A1- 2005 154 379
- US-A1- 2005 187 537

## Description

This invention relates to ophthalmic surgical equipment and more particularly to posterior segment ophthalmic surgical equipment.

### Background of the Invention

Microsurgical instruments typically are used by surgeons for removal of tissue from delicate and restricted spaces in the human body, particularly in surgery on the eye, and more particularly in procedures for removal of the vitreous body, blood, scar tissue, or the crystalline lens. Such instruments include a control console and a surgical handpiece with which the surgeon dissects and removes the tissue. With respect to posterior segment surgery, the handpiece may be a vitreous cutter probe, a laser probe, or an ultrasonic fragmenter for cutting or fragmenting the tissue and is connected to the control console by a long air-pressure (pneumatic) line and/or power cable, optical cable, or flexible tubes for supplying an infusion fluid to the surgical site and for withdrawing or aspirating fluid and cut/fragmented tissue from the site. The cutting, infusion, and aspiration functions of the handpiece are controlled by the remote control console that not only provides power for the surgical handpiece(s) (e.g., a reciprocating or rotating cutting blade or an ultrasonically vibrated needle), but also controls the flow of infusion fluid and provides a source of vacuum (relative to atmosphere) for the aspiration of fluid and cut/fragmented tissue. The functions of the console are controlled manually by the surgeon, usually by means of a foot-operated switch or proportional control.

During posterior segment surgery, the surgeon typically uses several handpieces or instruments during the procedure. This procedure requires that these instruments be inserted into, and removed out of the incision. This repeated removal and insertion can cause trauma to the eye at the incision site. To address this concern, hubbed cannulae were developed at least by the mid-1980s. These devices consist of a narrow tube with an attached hub. The tube is inserted into an incision in the eye up to the hub, which acts as a stop, preventing the tube from entering the eye completely. Surgical instruments can be inserted into the eye through the tube, and the tube protects the incision sidewall from repeated contact by the instruments. In addition, the surgeon can use the instrument, by manipulating the instrument when the instrument is inserted into the eye through the tube, to help position the eye during surgery.

Many surgical procedures require access to the sides or forward portion of the retina. In order to reach these areas, the surgical probes must be pre-bent or must be bendable intraoperatively. Articulating laser/illumination probes are known. See for example, USPN 5,281,214 (Wilkins, et al.). The articulation mechanism, however, adds extra complexity and expense. One flexible laser probe needing no articulation mechanism is commercially available, but this device uses a relatively large diameter optical fiber sheathed in a flexible tube comprising the distal tip, resulting in a large bend radius and large distal tip diameter with significant bend stiffness. These characteristics require that the distal tip contain a non-bent straight portion for ease of insertion of the bent portion, which must flexibly straighten as it passes through the hubbed cannula. The straight portion of the distal tip allows the bent portion to flexibly pass through the hubbed cannula before the distal cannula of the handpiece enters the hubbed cannula, to allow maximum bending clearance of the flexible portion, thereby minimizing the bending strain and corresponding frictional insertion forces. Such a large bend radius, large diameter flexible tube, and straight distal tip causes the useable portion of the fiber to extend a relatively long distance from the distal tip of the probe and limits access of the probe. A further disadvantage in the known art is the flexibility of the distal cannula, which is a function of the material properties and cross sectional moment of inertia, as determined by the gauge size of the outside diameter of the cannula to fit within the hubbed cannula, and the inside diameter of the cannula to accept the flexible tube. For any given material, the outer and inner diameters of the cannula determine the flexibility of the cannula. This flexibility limits the surgeon's ability to use the instrument to manipulate the position of the eye during surgery. Accordingly, a need continues to exist for a flexible-tip probe that does not require a straight portion of flexible tube at the distal tip, and which thus provides a more compact useable tip length, thereby allowing greater access to internal posterior structures of the eye without compromising insertion forces. The need also continues to exist for a flexible-tip probe which provides increased rigidity of the distal cannula to facilitate manipulation of the eye position during surgery.

US 2005187537 discloses a tube having a distal end portion made of a curved, flexible, shape retentive material such as superelastic, nickel-titanium memory metal alloy which has been heat-treated to retain a desired curved shape. The tube translates within and is constrained by a rigid sleeve, which may alternately be an instrument channel of an endoscope. When the distal end portion of the tube is extended from the sleeve, it returns to its original curved shape. Markings about the proximal and, or distal end portions of the tube enable the operator to know to what extent the distal end portion of the tube has been extended from the sleeve even when the distal end portion of the tube is not visible. The tube may be used for supporting and activating a cutting, abrading, coagulating, shrinking, or vaporizing device that is brought near or into contact with a tissue surface.

The state of the art is also represented by US-2005/0154379-A, EP-1,447,064-A, WO-2005/107664-A.

### Brief Summary of the Invention

The present invention improves upon prior art by providing a probe having a flexible, small diameter fiber within a flexible tube, comprising the distal tip of the probe, in accordance with claims which follow.

The small diameter fiber and tube combination allow the fiber to be bent in a tight radius along essentially the entire length of the exposed portion of the fiber, without the need for a straight portion to reduce insertion forces. Such a tight radius allows the fiber greater access to the internal posterior structures of the eye; thus increasing the treatment area of the probe, without compromising insertion forces.

Accordingly, an objective of the present invention is to provide a laser probe having a flexible, small diameter fiber/tube comprising the distal tip of the probe.

Another objective of the present invention is to provide a laser probe having a flexible, small diameter fiber/tube comprising the distal tip of the probe that is bent in a tight radius along essentially the entire length of the exposed portion of the fiber.

A further objective of the present invention is to provide a laser probe that allows greater access to the internal posterior structures of the eye. A further objective of the present invention is to provide increased rigidity of the distal cannula to facilitate manipulation of the eye position during surgery.

Other objectives, features and advantages of the present invention will become apparent with reference to the drawings, and the following description of the drawings and claims.

### Brief Description of the Drawings

FIG. 1 is a perspective view of the probe of the present invention.
FIG. 2 is an elevational view of the probe of the present invention.
FIG. 3 is a cross-sectional view of the probe of the present invention.

### Detailed Description of the Invention

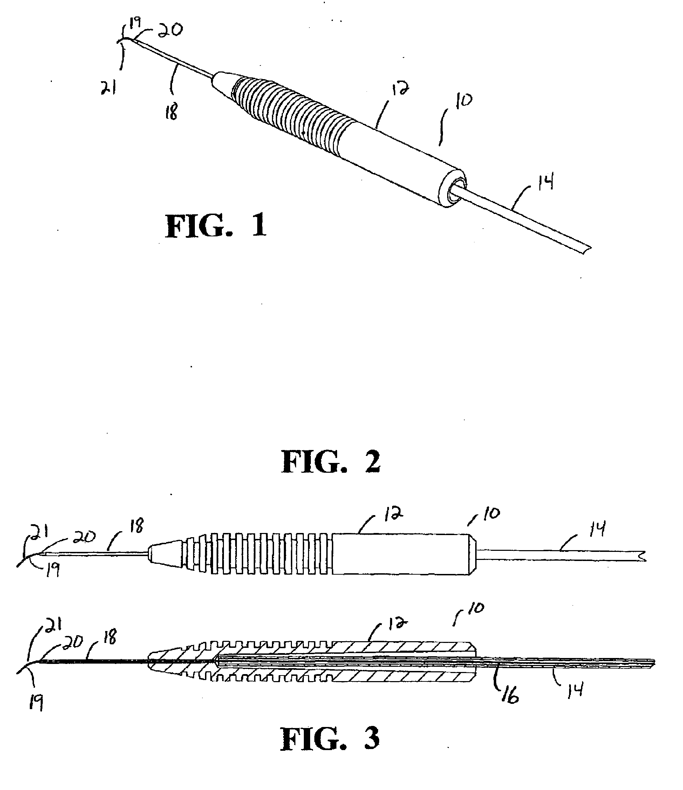

As best seen in the FIGS. 1, probe 10 of the present invention generally consists of handle or body 12, containing or encasing fiber optic 16, flexible tube 21, distal cannula 18, and fiber optic sheath 14. Body 12 is generally hollow and can be made from any suitable material such as stainless steel, titanium or thermoplastic. Cannula 18 may be made from any suitable material such as titanium or stainless steel and held within body 12 by any conventional method, such as adhesive or crimping. Fiber optic sheath 14 may be any suitable tubing such as thermoplastic or silicone. Fiber optic 16 is connected on a proximal end (not shown) to any suitable laser or illumination source through a connector of a type well-known in the art and is surrounded by flexible tube 21 with exposed portion 19. Flexible tube 21 is made from the shape memory alloy Nitinol, and is held within cannula 18 by any conventional method, such as adhesive or crimping, and encases fiber optic 16, which is held to inner diameter of flexible tube 21 by any conventional method such as adhesive or crimping. Fiber optic 16 and exposed section 19 of flexible tube 21 extend beyond distal end 20 of cannula 18 a distance of approximately 3 millimeters to 8 millimeters, with approximately 4 millimeters to 6 millimeters being most preferred. Fiber optic 16 may be any fiber optic material suitable for conducting laser of illumination light and preferable is silica (or glass) with an outer diameter of between 100µm and 125µm with at least exposed portion 19 within a 33 gauge (0.254 mm) OD flexible nitinol tube bent at an angle of approximately 45° on a radius of approximately between 4.5 millimeters and 6 millimeters along exposed section 19.

Importantly, exposed section 19 of fiber optic 16 is curved or bent beginning immediately at distal end 20 of cannula 18, with minimal or no straight section near distal end 20 of cannula 18. Such a construction improves peripheral access near the point of entry of cannula 18. By virtue of the smaller diameter flexible tube with significantly reduced cross sectional moment of inertia, the simultaneous insertion force of the exposed section 19 with the cannula 18 into a hubbed surgical cannula remains within an optimal range to facilitate manual insertion and extraction.

In use, exposed section 19 of fiber optic 16 can be straight so that exposed section 19 can be inserted into an eye through a 23 gauge (0.610 mm) or 25 gauge (0.508 mm) hubbed cannula. Once in the eye, the shape memory characteristics of the nitinol tube cause exposed section 19 to resume its curved configuration.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. A ophthalmic probe (10), comprising:
a) a generally hollow body (12);
b) a cannula (18) attached to the distal end of the body;
c) a fiber optic cable extending through the hollow body, the fiber optic cable having a fiber optic (16) extending through the cannula (18); and
d) an exposed portion (19) of the fiber optic, the exposed portion of the fiber optic extending beyond a distal end (20) of the cannula, the exposed portion of the fiber optic being encased in a nitinol tube (21) that is bent beginning immediately at the distal end (20) of the cannula (18) along a radius of between approximately 4.5 millimeters and 6.0 millimeters and the fiber optic (16) being fixedly held to the inner diameter of the nitinol tube;
**characterized by**
the nitinol tube (21) being fixedly held within the cannula (18).

2. The probe of claim 1, wherein the nitinol tube (21) is bent at an angle of approximately 45 degrees.

3. The probe of claim 1, wherein the fiber optic (16) has an outer diameter of between approximately 100 µm and 125 µm.

4. The probe of claim 1, wherein the exposed portion (19) extends beyond the distal end (20) of the cannula (18) a distance of approximately 3.0 millimeters to 8.0 millimeters.

5. The probe of claim 4, wherein the exposed portion extends beyond the distal end (20) of the cannula (18) a distance of approximately 4.0 millimeters to 6.0 millimeters.

## Patentansprüche

1. Augensonde (10), aufweisend:
a) einen im Wesentlichen hohlen Körper (12);
b) eine an dem distalen Ende des Körpers angebrachte Kanüle (18) ;
c) einen sich durch den hohlen Körper erstreckenden Lichtwellenleiter, wobei der Lichtwellenleiter eine sich durch die Kanüle (18) hindurch erstreckende Faseroptik (16) besitzt; und
d) einen ungeschützten Abschnitt (19) der Faseroptik, wobei sich der ungeschützte Abschnitt der Faseroptik über ein distales Ende (20) der Kanüle hinaus erstreckt, wobei der ungeschützte Abschnitt der Faseroptik in einem Nitinolröhrchen (21) eingeschlossen ist, das unmittelbar an dem distalen Ende (20) der Kanüle (18) beginnend entlang einem Radius zwischen angenähert 4,5 mm und 6,0 mm gebogen ist, und die Faseroptik (6) starr an dem Innendurchmesser des Nitinolröhrchens festgehalten wird;
**dadurch gekennzeichnet, dass**
das Nitinolröhrchen (21) starr in der Kanüle (18) festgehalten wird.

2. Sonde nach Anspruch 1, wobei das Nitinolröhrchen (21) in einem Winkel von annähernd 45 Grad gebogen ist.

3. Sonde nach Anspruch 1, wobei die Faseroptik (16) einen Durchmesser zwischen annähernd 100 µm und 125 µm hat.

4. Sonde nach Anspruch 1, wobei sich der ungeschützte Abschnitt (19) über das distale Ende (20) der Kanüle (18) über eine Strecke von angenähert 3,0 mm bis 8,0 mm hinaus erstreckt.

5. Sonde nach Anspruch 4, wobei sich der ungeschützte Abschnitt über das distale Ende (20) der Kanüle (18) über eine Strecke von angenähert 4,0 mm bis 6,0 mm hinaus erstreckt.

## Revendications

1. Sonde ophtalmique (10), comprenant :
a) un corps généralement creux (12) ;
b) une canule (18) fixée sur l'extrémité distale du corps ;
c) un premier câble de fibre optique s'étendant à travers le corps creux, le câble de fibre optique ayant une fibre optique (16) s'étendant à travers la canule (18) ; et
d) une partie exposée (19) de fibre optique, la partie exposée de fibre optique s'étendant au-delà d'une extrémité distale (20) de la canule, la partie exposée de la fibre optique étant enfermée dans un tube de Nitinol (21) qui est plié immédiatement à partir de l'extrémité distale (20) de la canule (18) le long d'un rayon compris entre approximativement 4,5 millimètres et 6,0 millimètres, et la fibre optique (16) étant maintenue de manière fixe sur le diamètre interne du tube en Nitinol ;
**caractérisée par** :
le tube en Nitinol (21) qui est maintenu de manière fixe à l'intérieur de la canule (18).

2. Sonde selon la revendication 1, dans laquelle le tube en Nitinol (21) est plié selon un angle d'approximativement 45 degrés.

3. Sonde selon la revendication 1, dans laquelle la fibre optique (16) a un diamètre externe compris entre approximativement 100 µm et 125 µm.

4. Sonde selon la revendication 1, dans laquelle la partie exposée (19) s'étend au-delà de l'extrémité distale (20) de la canule (18) sur une distance de l'ordre d'approximativement 3,0 millimètres à 8,0 millimètres.

5. Sonde selon la revendication 4, dans laquelle la partie exposée s'étend au-delà de l'extrémité distale (20) de la canule (18) sur une distance de l'ordre d'approximativement 4,0 millimètres à 6,0 millimètres.
